# EUROPEAN PATENT SPECIFICATION

(11) **EP 1 696 795 B1**
(45) Date of publication and mention of the grant of the patent: **29.04.2009**
(21) Application number: 03768098.0
(22) Date of filing: 04.11.2003
(51) Int. Cl.: A61B 5/15

(54) **PROCESS FOR THE PRODUCTION OF INTERCHANGEABLE VACUUM TEST TUBE HOLDERS FOR TAKING BLOOD SAMPLES AND PRODUCT OBTAINED THEREFROM**
VERFAHREN ZUR HERSTELLUNG VON HALTERN FÜR AUSWECHSELBARE EVAKUIERTE TESTRÖHRCHEN ZUR BLUTENTNAHME UND DAMIT HERGESTELLTE PRODUKTE
PROCEDE DE FABRICATION DE SUPPORTS DE TUBES A ESSAI INTERCHANGEABLES POUR PRELEVEMENTS SANGUINS ET PRODUIT AINSI OBTENU

(43) Date of publication of application: 06.09.2006
(73) Proprietor: Casarotto, Alfonsa, 45100 Rovigo (IT); Dall'Ara, Chiara, 35020 Masera (IT)
(72) Inventor: Casarotto, Alfonsa, 45100 Rovigo (IT); Dall'Ara, Chiara, 35020 Masera (IT)
(74) Representative: Vinci, Marcello
(86) International application number: PCT/IT2003/000713
(87) International publication number: WO 2005/041773

(56) References cited:
- EP-A- 0 732 077
- US-A- 3 326 206
- US-A- 5 188 119

## Description

This patent concerns devices for taking blood samples and in particular it concerns the holders provided with needle, for veins or arteries, in which the vacuum test tubes for taking blood samples are inserted and the process for their production.

Many medical diagnoses require the analysis of blood samples which are taken directly from the patient's vein.

When blood samples are taken, it is very important to prevent the transmission, both between patients and between patient and nurse, of any diseases, germs, viruses and similar.

The nurse must not insert the needle through the vein, penetrating it from side to side, because this produces a haematoma in the patient and also because it makes it impossible to take the blood sample.

The syringe, as a means of taking blood samples from the patient, has been replaced by a holder provided with needle, integral with it, and various vacuum test tubes that are inserted in said holder each time.

The holder consists of a plastic cylinder with the rear end open and the front end closed by a circular wall through which the needle passes.

Said cylinder, which is suited to contain the vacuum test tube, has the disadvantage of being very wide.

Part of the needle outside the holder is inserted in the vein from which the blood sample is to be taken, while the tip of the needle which is housed inside the holder pierces the seal cap of the vacuum test tube inserted in the holder, thus conveying the blood inside said vacuum test tube.

The holders with needle with two opposite tips currently known have the needle positioned centrally, or coaxially, with respect to the holder.

During the taking of blood samples, when the needle of the holder is inserted in the patient's vein, the needle penetrates the skin at a very wide angle, as the thickness of the holder makes it impossible to position the needle near the skin in a substantially tangential position. The nurse is therefore obliged to contrive various solutions, such as raising the patient's skin or pressing the holder hard against the patient's skin. All this does not prevent possible perforation of the vein, however.

Furthermore, said contrivances cause discomfort and in some cases even pain to the patient.

Since the tip of the needle is slanted or lance-shaped, for better insertion in the skin and in the vein, each time the nurse must check the position of the tip of the needle, which must have its oblique side facing upwards for correct insertion into the skin and vein.

Some nurses use butterfly needles, i.e. small needles provided at the sides with two flat flexible grips and provided with a connection tube for the support or for the syringe. These butterfly needles simplify insertion into the vein and make the blood sampling less painful, but the nurse has to prepare the butterfly needle, connect it to the support or syringe, insert the butterfly needle into the vein and keep it in position with her/his hand or a plaster throughout the sampling operation.

Furthermore, butterfly needles are expensive to purchase and dispose of.

Holders exist that are provided with non-central needle, i.e. a needle positioned near the side wall of the holder and parallel to it.

These holders are described in the patent US 5938622.

Although these holders theoretically permit better insertion of the needle into the vein, they are expensive and difficult to produce. These vacuum test tubes, in addition to being expensive to purchase, must be inserted correctly in the holder, i.e. with the eccentric pierceable area perfectly aligned with the needle inside the holder.

Holders with the needle bent at two points, i.e. roughly S-shaped with the two end parts parallel and the intermediate part slanting, are also known.

The part of the needle inside the holder is generally central to or coaxial with the holder, whereas the outer part of the needle is eccentric and out of line.

Said holders with needle bent at two points are described in patents US 3520292 and 3326206, for example.

The production of said holders with needle bent at two points may require the pre-bending of the needle, the creation around its slanting part of the circular wall which constitutes the bottom of the holder and, lastly, the creation of the cylindrical wall of the holder.

All said work phases involve lengthy production times, the need to position and align the various parts, the possibility of inaccuracy and imperfect joining of the various parts.

Consequently, the production of said holders with bent needle is very costly.

Said holders with bent needle are not convenient for use, also due to the fact that in practice their production process does not allow the outer part of the needle to be positioned in correspondence with the outer edge of the casing. Said distance of the needle from the edge of the casing, albeit shorter than the distance existing in the holders with central needle, limits the insertion of the needle into the vein and all the sampling operations.

In order to eliminate the above-mentioned disadvantages, a new process has been designed for the production of holders with needle bent at two points which can be used with interchangeable vacuum test tubes for taking blood samples.

The aim of the new process is to produce a holder with needle bent at two points in only a few simple stages.

A further aim of the new process is to produce a holder with needle bent at two points having the outer part of the needle practically aligned with the outer edge of the casing, that is, with a much narrower or nil penetration angle.

A further aim of the new process is to produce a holder with needle bent at two points without the possibility of inaccuracy, imperfections, non-alignments or incorrect orientation.

The aim of the new holder with needle bent at two points is to facilitate the insertion of the holder needle into the vein without causing the patient discomfort or pain.

A further aim of the new holder is to facilitate the insertion of the holder needle into the vein with no need for the nurse to check the position of the tip of the needle.

A further aim of the new holder is to facilitate the insertion of the holder needle into the vein with no need for the nurse to raise the patient's skin.

A further aim of the new holder is to facilitate the insertion of the holder needle into the vein, avoiding the risk of perforating the vein from side to side.

A further aim of the new holder is to permit the use of common vacuum test tubes provided with seal cap suitable for central perforation.

These and other direct and complementary aims are achieved through the implementation of the new process for the production of holders with needle bent at two points which can be used with interchangeable vacuum test tubes for taking blood samples and through the product obtained therefrom. The new holder, which is preferably of the disposable type, comprises a generically cylindrical casing closed at one end, designed to receive vacuum test tubes with seal cap for central perforation, and a shaped needle, preferably consisting of two parallel straight sections not lying on the same axis, joined by a further slanting or generically S-shaped section, said needle being joined to the casing so that its portion inside the casing is coaxial with said casing and so that its portion outside the casing and facing the same is shaped and, in its end part, parallel and eccentric to the axis of the casing, while an outer support wall or front connection wall acts as a thrust bearing for the needle.

The casing features a wall designed to house and join the angled, bent or shaped section of the needle to the casing.

The holder also has the advantage of being disposable, thus avoiding all possible risks for the users.

The process for the production of the new holder with needle bent at two points includes the complete production of the casing with connection wall, the insertion of the needle still straight and with the angled ends correctly oriented into the end circular wall of the casing, the fixing of said needle to said circular wall, the double bending of the needle until it adheres to the slanting connection wall and is aligned with the outer edge of the casing and the final fixing of the needle to the end of the slanting connection wall.

Said connection wall or outer support wall can be used as a grip for the new holder.

The characteristics of the new process for the production of the new holder with needle bent at two points and of the product obtained therefrom will be better illustrated by the following description, with reference to the drawings attached as a non-restrictive example.

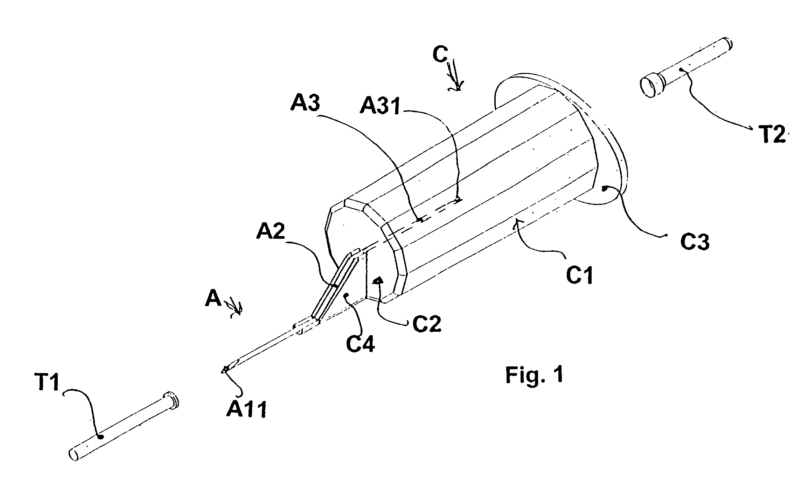

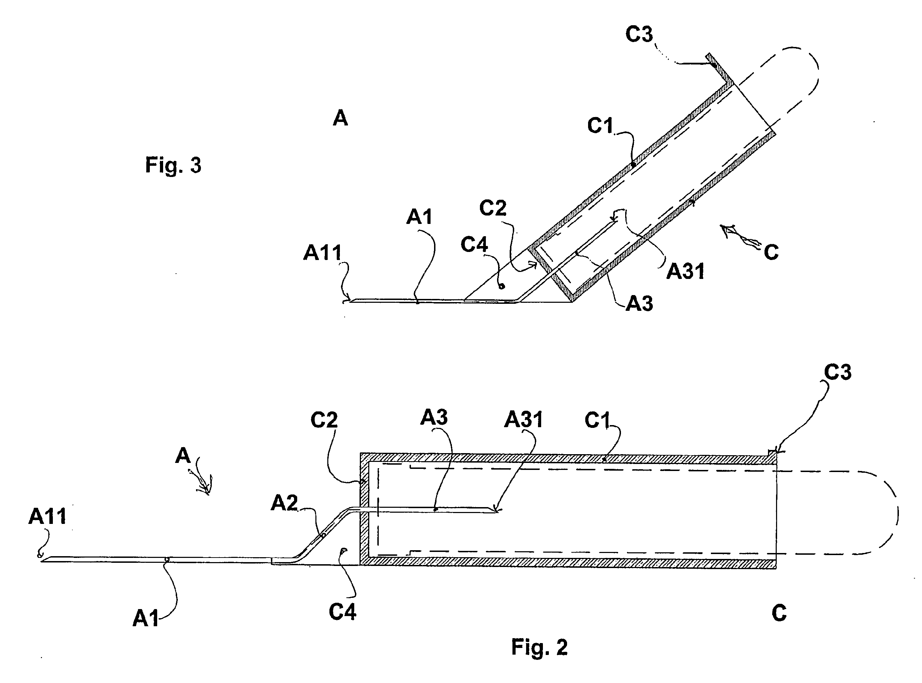

Figure 1 shows an axonometric view of the new holder, comprising a casing (C) and a shaped needle (A). Figure 2 shows a cross section of said holder.

The shaped needle (A) consists of three consecutive sections (A1, A2, A3), of which the two extreme sections (A1 , A3) are parallel and not lying on the same axis. The intermediate section (A2) connects said two extreme sections (A1, A3) and is slanting with respect to each of them.

Both said extreme sections (A1, A3) have angled or slanting tip (A11, A31), in order to permit the penetration into the skin and vein and the perforation of and penetration into the seal cap of a vacuum test tube, respectively.

The casing (C) consists of a cylinder (C1) closed on the front side (C2) and provided with outer tabs (C3) at the other end.

On the front closing wall (C2) of the casing (C) the shaped needle (A) is applied, so that an end section (A3) of said needle (A) is inside the casing (C), coaxial with the cylindrical part (C1) of the casing (C).

The other end (A1) of the shaped needle (A) is outside the casing (C) and substantially aligned with the lower outer cylindrical surface of the casing (C).

Due to the position of the shaped needle (A) with respect to the casing (C), its intermediate section (A2) is outside the casing (C) and facing the front closing wall (C2) of the casing (C).

The tip (A1) of the needle (A) that is outside the casing (C) has the angled, or slanting side (A11) facing upwards or facing the centre or the axis of the casing (C).

The casing (C) features a connection wall (C4) designed to house and connect the intermediate section (A2) of the needle (A) with the casing (C). The lower edge of said connection wall (C4) is parallel to the last outer section (A1) of the needle (A) and provides a sliding surface for the holder (C) during the introduction of the needle (A).

The contact surface between said connection wall (C4) and the needle (A), in particular the intermediate slanting section (A2) of the needle (A), is generically semicircular, preferably with a slight undercut, in order to correctly house and retain the needle (A).

This connection or support wall (C4) can be used as a grip for the new holder.

Two caps (T1, T2) are provided, which can be applied to protect and cover the end sections (A1) and (A3) and the two tips (A11, A31) of the needle (A).

The casing (C), or part of it, can bear the colour of the needle gauge international code.

Figure 3 shows in section a possible variant of the invention, in which the needle (A) is bent in two parts (A1, A3), of which an end part (A3) of the needle (A) is included inside the casing (C) and is coaxial with it, while the other end part (A1) of the needle (A) is outside the casing (C) and slanting with respect to said casing (C).

The process for the production of said new holder with needle bent at two points comprises only a few simple stages.

Initially the whole casing (C) is produced, preferably by moulding of thermoplastic material, with the outer tabs (C3) and with the slanting connection wall (C4). The hole housing the needle (A) is provided on the front wall (C2).

The needle (A), linear and not bent, is inserted in said hole of said front wall (C2) of the casing, in such a way as to correctly position its end section (A3) inside the casing.

In this insertion phase, the needle (A) is already positioned with the slanting tip (A11) correctly arranged, in particular facing upwards with respect to the connection wall (C4).

At this point the needle (A) is fixed to the front wall (C2) of the casing (C), for example by means of glue.

Subsequently the needle (A) is bent twice: the first operation bends the needle (A) so that it adheres to the connection wall (C4), and in particular positioning it in the semicircular housing of said wall (C4); the second bending operation, at the end of the connection wall (C4), aligns the end section (A1) of the needle (A) with the outer surface of the casing (C) and with the sliding surface of the connection wall (C4).

Lastly, the two sections (A1, A2) of the needle (A) outside the casing (C) are fixed to the connection wall (C4), preferably by means of glue, in correspondence with the end of the connection wall (C4) opposite the casing (C).

The method described above for the production of the new holder with needle bent at two points requires the simple moulding of the casing (C) and the preparation of the needle (A) straight with angled tip (A11), the insertion of the needle (A) into the casing (C) and, lastly, the bending and joining of the needle (A) to the casing (C) and to the connection wall (C4).

The new holder constituted and produced as described above offers considerable advantages.

After removing the cap (T1) from the needle (A), the nurse grips the new holder by its cylindrical body (C1) or the wall or connection wall (C4), leaving the outer section (A1) of the needle (A) facing downwards. Consequently, the new holder in the nurse's hand is already in the correct position for use, with the tip (A11) of the needle (A) already correctly facing upwards with respect to the patient's skin and vein.

The eccentric position of the needle (A1) with respect to the casing (C), and in particular its position aligned with the outer surface of the casing (C), allows the nurse to insert the needle (A) into the skin and vein with minimum penetration angle.

The new holder, with its greatly reduced or nil penetration angle, significantly reduces the discomfort or pain caused to the patient and avoids possible vein perforation errors.

The new holder, with its greatly reduced penetration angle, facilitates the insertion of the needle (A) into the vein.

The new holder with the eccentric needle (A) allows the nurse to correctly insert the needle (A) into the vein with no need to take any particular precautions, such as raising the patient's skin or varying the penetration angle during penetration.

The reduced penetration angle of the new holder considerably reduces the risk of perforating the vein from side to side.

The new holder, having the needle section (A3) inside the casing (C) positioned coaxially with the casing itself, permits the use of the common and widespread vacuum test tubes with seal cap for central perforation.

Therefore, with reference to the preceding description and the attached drawings, the following claims are put forth.

## Claims

1. Process for the production of holders for vacuum test tubes, comprising a cylindrical casing (C), designed to house said vacuum test tube for blood sampling, provided with a needle (A) partially housed in said casing (C), with an inner part (A3) of the needle (A) generically coaxial with the casing (C), the process comprising:
■ the production of the whole casing (C) by moulding of thermoplastic material, with a hole suitable for housing the needle (A) on a cloning front wall (C2) of the casing and with a support wall or lower connection wall (C4) of the casing generically triangular in shape and having one side adhering to the front wall (C2) between said hole and an outer cylindrical wall of the casing (C) and one lower side aligned with said cylindrical wall of the casing (C);
■ the insertion of the needle (A) straight, with an oblique cut of the tip (A11) facing upwards into said hole in the front wall (C2) of the casing (C) and the fixing of said needle (A) in correspondence with the front wall (C2), in such a way as to house the inner part (A3) of the needle (A) inside the casing (C);
■ a first bending of the needle (A) to slant it with respect to the axis of the casing (C) and make it adhere to a slanting side of the connection wall (C4);
■ a further bending of an outer part (A1) of the needle (A) to align said outer part (A1) of the needle (A) with the outer cylindrical wall of the casing (C);
■ the fixing of the needle (A) to the support wall or lower connection wall (C4), preferably on the end of said support wall or lower connection wall (C4) opposite the casing (C).

2. Holder to be used with interchangeable vacuum test tubes for taking blood samples, comprising a generically cylindrical casing (C), the whole casing being produced by moulding of thermoplastic material and having a closing front wall (C2) at one end, provided with a needle (A) bent in three parts, of which the two end parts (A1, A3) are parallel, not lying on the same axis, and the intermediate part (A2), connecting said two parts (A1, A3), is external to said casing (C) and is slanting with respect to the casing, and wherein said needle (A) is fixed to the front wall (C2) of the casing (C), so that an end part (A3) of the needle (A) is inside the casing (C) and is coaxial with it, and wherein the other end part (A1) of the needle is outside the casing (C) and generically aligned with an outer cyclindrical wall of the casing (C), and wherein said casing (C) comprises on its front wall (C2) a support wall or lower connection wall (C4) designed to house the intermediate part (A2) of the needle (A) and to connect it with the casing (C), said support wall or lower connection wall providing a lower sliding surface for the holder during the introduction of the needle (A).

3. Holder for interchangeable vacuum test tubes for taking blood samples, comprising a generally cylindrical casing (C), the whole casing being produced by moulding of thermoplastic material and being a closing front wall (C2) at one end, provided with a needle (A) bent in two parts (A1, A3), and wherein said needle (A) is fixed to said front wall (C2) of the casing (C), so that a part (A3) of the needle (A) is inside the casing (C) and is coaxial with it, and wherein the other part (A1) of the needle (A) is outside the casing (C) and slanting with respect to the casing (C), and wherein said casing (C) comprises on its front wall (C2) a support wall or lower connection wall (C4) designed to house the outer part (A1) of the needle (A) and to connect it with the casing (C), said support wall or lower connection wall providing a lower sliding surface for the holder during the introduction of the needle (A).

4. Holder for interchangeable vacuum test tubes for taking blood samples according to claim 2 or claim 3, **characterized in that** the connection wall (C4) of the casing is provided with a semicircular seat suitable for housing and fixing the needle (A).

5. Holder for interchangeable vacuum test tubes for taking blood samples according to any of claims 2 to 4, **characterized in that** the outer tip (A1) of the needle (A) has an oblique cut (A11) facing upwards.

6. Holder for interchangeable vacuum test tubes for taking blood samples according to any of claims 2 to 5, **characterized in that** it is provided with at least one cap (T1) that can be applied to protect and cover the tip (A11) of the needle (A).

7. Holder for interchangeable vacuum test tubes for taking blood samples according to any of claims 2-6, **characterized in that** the surface of the casing (C) bears a colour and/or wording indicating the international code of the needle gauge.

## Patentansprüche

1. Verfahren zur Herstellung von Haltern für Vakuum-Probenröhrchen, umfassend ein zylindrisches Gehäuse (C), das dazu vorgesehen ist, das Vakuum-Probenröhrchen für eine Blutprobennahme aufzunehmen, und eine Nadel (A) aufweist, die teilweise im Gehäuse (C) aufgenommen ist, wobei ein innerer Abschnitt (A3) der Nadel (A) im Allgemeinen koaxial zum Gehäuse (C) ist, wobei das Verfahren Folgendes umfasst:
■ das Herstellen des kompletten Gehäuses (C), durch Formen eines thermoplastischen Materials, mit einem zur Aufnahme der Nadel (A) geeigneten Loch an einer vorderen Verschlusswand (C2) des Gehäuses und mit einer Stützwand oder unteren Verbindungswand (C4) des Gehäuses von im Allgemeinen dreieckiger Form, wobei eine Seite dieser an der Vorderwand (C2) zwischen dem Loch und einer zylindrischen Außenwand des Gehäuses (C) anliegt und eine untere Seite mit der zylindrischen Wand des Gehäuses (C) fluchtend ausgerichtet ist;
■ das Einführen der Nadel (A), wobei ein schräger Schnitt der Spitze (A11) nach oben weist, gerade in das Loch in der Vorderwand (C2) des Gehäuses (C) und das Befestigen der Nadel (A) an der Vorderwand (C2), sodass der innere Abschnitt (A3) der Nadel (A) im Inneren des Gehäuses (C) aufgenommen ist;
■ ein erstes Biegen der Nadel (A), um sie in Bezug auf die Achse des Gehäuses (C) zu neigen und sie dazu zu bringen, an einer geneigten Seite der Verbindungswand (C4) anzuliegen ;
■ ein weiteres Biegen eines äußeren Abschnitts (A1) der Nadel (A), um den äußeren Abschnitt (A1) der Nadel (A) mit der zylindrischen Außenwand des Gehäuses (C) fluchtend auszurichten;
■ das Befestigen der Nadel (A) an der Stützwand oder unteren Verbindungswand (C4), vorzugsweise an dem Ende der Stützwand oder unteren Verbindungswand (C4), das dem Gehäuse (C) gegenüberliegt.

2. Halter zur Verwendung mit austauschbaren Vakuum-Probenröhrchen zur Blutprobennahme, umfassend ein im Allgemeinen zylindrisches Gehäuse (C), wobei das gesamte Gehäuse durch Formen eines thermoplastischen Materials hergestellt ist, an einem Ende eine vordere Verschlusswand (C2) aufweist und mit einer Nadel (A) ausgestattet ist, die zu drei Abschnitten gebogen ist, von denen die beiden Endabschnitte (A1, A3) parallel sind und nicht auf derselben Achse liegen und der Zwischenabschnitt (A2), der die beiden Abschnitte (A1, A3) verbindet, außerhalb des Gehäuses (C) angeordnet ist und in Bezug auf das Gehäuse geneigt ist, wobei die Nadel (A) an der Vorderwand (C2) des Gehäuses (C) befestigt ist, sodass ein Endabschnitt (A3) der Nadel (A) im Inneren des Gehäuses (C) angeordnet ist und zu diesem koaxial ist, wobei der andere Endabschnitt (A1) der Nadel außerhalb des Gehäuses (C) angeordnet ist und im Allgemeinen mit einer zylindrischen Außenwand des Gehäuses (C) fluchtend ausgerichtet ist, wobei das Gehäuse (C) an seiner Vorderwand (C2) eine Stützwand oder untere Verbindungswand (C4) aufweist, die dazu vorgesehen ist, den Zwischenabschnitt (A2) der Nadel (A) aufzunehmen und mit dem Gehäuse (C) zu verbinden, wobei die Stützwand oder untere Verbindungswand während des Einführens der Nadel (A) eine untere Gleitoberfläche für den Halter bildet.

3. Halter für austauschbare Vakuum-Probenröhrchen zur Blutprobennahme, umfassend ein im Allgemeinen zylindrisches Gehäuse (C), wobei das gesamte Gehäuse durch Formen eines thermoplastischen Materials hergestellt ist, an einem Ende eine vordere Verschlusswand (C2) aufweist und mit einer Nadel (A) ausgestattet ist, die zu zwei Abschnitten (A1, A3) gebogen ist, wobei die Nadel (A) an der Vorderwand (C2) des Gehäuses (C) befestigt ist, sodass ein Abschnitt (A3) der Nadel (A) im Inneren des Gehäuses (C) angeordnet ist und zu diesem koaxial ist, wobei der andere Abschnitt (A1) der Nadel (A) außerhalb des Gehäuses (C) angeordnet ist und in Bezug auf das Gehäuse (C) geneigt ist, wobei das Gehäuse (C) an seiner Vorderwand (C2) eine Stützwand oder untere Verbindungswand (C4) aufweist, die dazu vorgesehen ist, den äußeren Abschnitt (A1) der Nadel (A) aufzunehmen und mit dem Gehäuse (C) zu verbinden, wobei die Stützwand oder untere Verbindungswand während des Einführens der Nadel (A) eine untere Gleitoberfläche für den Halter bildet.

4. Halter für austauschbare Vakuum-Probenröhrchen zur Blutprobennahme nach Anspruch 2 oder 3, **dadurch gekennzeichnet, dass** die Verbindungswand (C4) des Gehäuses mit einem halbkreisförmigen Sitz ausgestattet ist, der dazu geeignet ist, die Nadel (A) aufzunehmen und zu befestigen.

5. Halter für austauschbare Vakuum-Probenröhrchen zur Blutprobennahme nach einem der Ansprüche 2 bis 4, **dadurch gekennzeichnet, dass** die äußere Spitze (A1) der Nadel (A) einen schrägen Schnitt (A11) aufweist, der nach oben weist.

6. Halter für austauschbare Vakuum-Probenröhrchen zur Blutprobennahme nach einem der Ansprüche 2 bis 5, **dadurch gekennzeichnet, dass** er mit mindestens einer Kappe (T1) ausgestattet ist, die aufgesetzt werden kann, um die Spitze (A11) der Nadel (A) zu schützen und zu bedecken.

7. Halter für austauschbare Vakuum-Probenröhrchen zur Blutprobennahme nach einem der Ansprüche 2 bis 6, **dadurch gekennzeichnet, dass** die Oberfläche des Gehäuses (C) eine Farbe und/oder einen Schriftzug trägt, die/der den internationalen Code für den Gauge-Wert der Nadel angibt.

## Revendications

1. Procédé pour la fabrication de supports pour tubes à essai sous vide comportant une enveloppe cylindrique (C), destinée à accueillir ledit tube à essai sous vide pour prélèvements sanguins, munie d'une aiguille (A) partiellement logée dans ladite enveloppe (C) avec une partie interne (A3) de l'aiguille (A) est généralement coaxiale à l'enveloppe (C), ledit procédé comportant :
- la fabrication de l'enveloppe (C) dans son ensemble par moulage d'une matière thermoplastique avec un trou apte à accueillir l'aiguille (A) dans une paroi avant de fermeture (C2) de l'enveloppe et avec une paroi de soutien ou une paroi inférieure de liaison (C4) de l'enveloppe ayant généralement une forme triangulaire et un côté adhérant à la paroi avant (C2) entre ledit trou et une paroi cylindrique externe de l'enveloppe (C) et un côté inférieur aligné sur ladite paroi cylindrique de l'enveloppe (C) ;
- l'insertion de l'aiguille (A) droite avec une entaille oblique de la pointe (A11) orientée vers le haut dans ledit trou dans la paroi avant (C2) de l'enveloppe (C) et la fixation de ladite aiguille (A) à hauteur de la paroi avant (C2) de façon à accueillir la partie interne (A3) de l'aiguille (A) à l'intérieur de l'enveloppe (C) ;
- un premier pliage de l'aiguille (A) afin de l'incliner par rapport à l'axe de l'enveloppe (C) et de la faire adhérer à un côté incliné de la paroi de liaison (C4) ;
- un deuxième pliage d'une partie externe (A1) de l'aiguille (A) afin d'aligner ladite partie externe (A1) de l'aiguille (A) sur la paroi cylindrique externe de l'enveloppe (C) ;
- la fixation de l'aiguille (A) à la paroi de soutien ou à la paroi inférieure de liaison (C4), préférablement à l'extrémité de ladite paroi de soutien ou de ladite paroi inférieure de liaison (C4) opposée à l'enveloppe (C).

2. Support à utiliser avec des tubes à essai sous vide interchangeables pour prélèvements sanguins, comportant une enveloppe généralement cylindrique (C), l'enveloppe dans son ensemble étant fabriquée par moulage d'une matière thermoplastique et ayant une paroi avant de fermeture (C2) à une extrémité, munie d'une aiguille (A) pliée en trois parties, dont les deux parties terminales (A1, A3) sont parallèles et ne sont pas sur le même axe, tandis que la partie intermédiaire (A2), reliant lesdites deux parties terminales (A1, A3), se trouve à l'extérieur de ladite enveloppe (C) et est inclinée par rapport à celle-ci, où ladite aiguille (A) est fixée sur la paroi avant (C2) de l'enveloppe (C), de façon à ce que la partie terminale (A3) de l'aiguille (A) se trouve à l'intérieur de l'enveloppe (C) et est coaxiale à celle-ci, tandis que l'autre partie terminale (A1) de l'aiguille se trouve à l'extérieur de l'enveloppe (C) et est généralement alignée sur une paroi cylindrique externe de l'enveloppe (C), et où ladite enveloppe (C) comporte sur la paroi avant (C2) une paroi de soutien ou une paroi inférieure de liaison (C4) apte à accueillir la partie intermédiaire (A2) de l'aiguille (A) et à la relier avec l'enveloppe (C), ladite paroi de soutien ou ladite paroi inférieure de liaison offrant au support une surface inférieure de glissement basse pendant l'introduction de l'aiguille (A).

3. Support à utiliser avec des tubes à essai sous vide interchangeables pour prélèvements sanguins, comportant une enveloppe généralement cylindrique (C), l'enveloppe dans son ensemble étant fabriquée par moulage d'une matière thermoplastique et ayant une paroi avant de fermeture (C2) à une extrémité, ledit support étant muni d'une aiguille (A) pliée en deux parties (A1, A3), **caractérisé en ce que** ladite aiguille (A) est fixée à ladite paroi avant (C2) de l'enveloppe (C), de façon à ce que une partie (A3) de l'aiguille (A) se trouve à l'intérieur de l'enveloppe (C) et est coaxiale à celle-ci, tandis que l'autre partie (A1) de l'aiguille (A) se trouve à l'extérieur de l'enveloppe (C) et est inclinée par rapport à celle-ci, ainsi que **caractérisé en ce que** ladite enveloppe (C) comporte sur la paroi avant (C2) une paroi de soutien ou une paroi inférieure de liaison (C4) apte à accueillir la partie externe (A1) de l'aiguille (A) et à la relier avec l'enveloppe (C), ladite paroi de soutien ou ladite paroi inférieure de liaison offrant au support une surface de glissement basse pendant l'introduction de l'aiguille (A).

4. Support à utiliser avec des tubes à essai sous vide interchangeables pour prélèvements sanguins selon la revendication 2 ou la revendication 3, **caractérisé en ce que** la paroi de liaison (C4) de l'enveloppe est munie d'un logement semi-circulaire apte à accueillir et à fixer l'aiguille (A).

5. Support à utiliser avec des tubes à essai sous vide interchangeables pour prélèvements sanguins selon les revendications de 2 à 4, **caractérisé en ce que** la pointe externe (A1) de l'aiguille (A) présente une entaille oblique (A11) orientée vers le haut.

6. Support à utiliser avec des tubes à essai sous vide interchangeables pour prélèvements sanguins selon l'une quelconque des revendications de 2 à 5, **caractérisé en ce qu'**il comporte au moins un capuchon (T1) à insérer sur la pointe (A11) de l'aiguille (A) afin de la protéger et de la couvrir.

7. Support à utiliser avec des tubes à essai sous vide interchangeables pour prélèvements sanguins selon l'une quelconque des revendications de 2 à 6, **caractérisé en ce que** la surface de l'enveloppe (C) présente une couleur et/ou porte la mention indiquant le code international du calibre de l'aiguille.
